# EUROPEAN PATENT SPECIFICATION

(11) **EP 1 855 762 B1**
(45) Date of publication and mention of the grant of the patent: **30.05.2012**
(21) Application number: 05784280.9
(22) Date of filing: 05.03.2005
(51) Int. Cl.: A61Q 19/08, A61K 8/19

(54) **COSMETIC COMPOSITION ENRICHED WITH 1H216O**
MIT 1H216O ANGEREICHTERTE KOSMETISCHE ZUSAMMENSETZUNG
COMPOSITION COSMETIQUE ENRICHIE EN 1H216O

(43) Date of publication of application: 21.11.2007
(73) Proprietor: Soloviev, Sergey Pavlovich, Moscow 115432 (RU); Timantti AB, 104 30 Stockholm (SE)
(72) Inventor: Soloviev, Sergey Pavlovich, Moscow 115432 (RU)
(74) Representative: Hayes, Adrian Chetwynd
(86) International application number: PCT/RU2005/000100
(87) International publication number: WO 2006/096082

(56) References cited:
- WO-A-2005/070438
- US-A- 5 788 953
- US-B1- 6 461 583
- DATABASE WPI Section Ch, Week 200445 Derwent Publications Ltd., London, GB; Class B06, AN 2004-472144 XP002351736 & JP 2004 175775 A (INT SCI KK) 24 June 2004 (2004-06-24)
- DATABASE WPI Section Ch, Week 200459 Derwent Publications Ltd., London, GB; Class B06, AN 2004-607408 XP002351737 & JP 2004 231627 A (INT SCI KK) 19 August 2004 (2004-08-19)
- DATABASE WPI Section Ch, Week 200448 Derwent Publications Ltd., London, GB; Class B06, AN 2004-503136 XP002351738 & JP 2004 189708 A (INT SCI KK) 8 July 2004 (2004-07-08)

## Description

### Technical Field

The present invention relates to cosmetic compositions production. More specifically, this invention relates to production of cosmetic compositions enriched with ¹H₂¹⁶O by weight of water of cosmetic compositions, in other words cosmetic compositions with increased ¹H₂¹⁶O content in comparison with typical cosmetic compositions.

### Background of the Invention

The skin is the largest organ in the body. The skin protects the deeper tissues from injury, drying, and invasion by foreign organisms. The skin contains the peripheral endings of many sensory nerves, as well as blood vessels. It plays an important part in regulating body temperature and also has limited excretory and absorbing powers. The skin is the first line of defense of the immune system.

At the same time, the human skin shows the most visible signs of aging and also visible damages or disturbances such as wrinkles, increased skin pore dimensions, nevus's pigmentosis, skin sulci and other tissue changes connect with the intensive influence external and internal factors.

Therefore the problem of how to improve the health and appearance of human skin is ubiquitous. Searching for potentialities and methods to increasing of effectiveness and quality cosmetic compositions for maintain skin nutrition and appearance is an important.

In the main current cosmetic remedies are complicated multicomponent compositions. Water is one of the necessary ingredients in cosmetic composition as it is a universal and the most physiologically acceptable solvent.

Any water is a composition of water as a chemical agent and some other substances such as suspension particles, chemical and biological admixtures and so on. Water purification methods depend on subsequent use of water and can be different, such as filtration, distillation, reverse osmosis and so on. The traditional water purification methods are able to eliminate only admixtures from water and have no effect on water as a chemical agent.

Any water as a chemical agent is a composition of 9 isotope varieties of water molecule such as: ¹H₂¹⁶O, ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O formed by stable isotopes of hydrogen - ¹H, ²H, and stable isotopes of oxygen - ¹⁶O, ¹⁷O, ¹⁸O. The other name for these isotope varieties of water molecule is isotopologues.

The term "isotopologue" is defined in accordance with IUPAC Compendium of Chemical Terminology 2nd Edition (1997) and refers to a molecular entity that differs only in isotopic composition (number of isotopic substitutions), e.g. ¹H₂¹⁶O, ¹H²H¹⁶O, ¹H₂¹⁸O. Herein and after the terms «isotope variety of water molecules» and «isotopologue» are used as convertible terms.

The content of water isotopologues in Ocean Water is stated as the internationally accepted water standard VSMOW. In Ocean Water the level of ¹H₂¹⁶O molecules comprising light isotopes ¹H and ¹⁶O is 99.731% (Vienna Standard Mean Ocean Water, VSMOW), and about 0.2683% of the Ocean Water is formed by water molecules comprising heavy isotopes ²H, ¹⁷O, ¹⁸O (0.0372% ¹H₂¹⁷O, 0.199983% ¹H₂¹⁸O, 0.031069% ¹H²H ¹⁶O, and etc.) (Rothman et al., J. Quant. Spectrosc. Radiat. Transfer, 1998, 60, 665. Rothman et al., J. Quant. Spectrosc. Radiat. Transfer, 2003, 82, p.9). The abundance of water isotopologues in natural water varies depending on Earth regions and climate conditions and is typically expressed as the deviation, δ, relative to the VSMOW standard. The natural water with maximum content of light water isotopologue ¹H₂¹⁶O was found in Antarctica (Standard Light Antarctic Precipitation, SLAP), wherein said δ-values of residual heavy isotopes are δ²H = -415.5‰, δ¹⁷O = -28.1‰, and δ¹⁸O = -53.9‰ that corresponds to the 99.757 % level of light water isotopologue ¹H₂¹⁶O (R. van Trigt, Laser Spectrometry for Stable Isotope Analysis of Water Biomedical and Paleoclimatological Applications, 2002, Groningen: University Library Groningen, p. 50). In other words, in nature the less concentration of molecules, comprising ²H, ¹⁷O, ¹⁸O heavy isotopes, was found in Antarctica and corresponds to the 99.757 % level of light water isotopologue ¹H₂¹⁶O.

Thus, natural water with the abundance of light water isotopologue ¹H₂¹⁶O more than 99.757% is not found in nature.

In typical natural water the residual concentration of the molecules, comprising ²H, ¹⁷O, ¹⁸O heavy isotopes, such as ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O can amount to 2.97 g/l.

Since total levels of deuterium-comprising isotopologues in water is rather more 0.3 g/l (0.031%) (Rothman et al., J. Quant. Spectrosc. Radiat. Transfer, 1998, 60, 665. Rothman et al., J. Quant. Spectrosc. Radiat. Transfer, 2003, 82, p.9) a complete depletion of natural water of deuterium-comprising isotopologues provides water with the level of light water isotopologue ¹H₂¹⁶O no more than 99.76%.

Thus, water with abundance of light water isotopologue ¹H₂¹⁶O more than 99.76% is unknown from the art.

Method and apparatus for production of the water with abundance of ¹H₂¹⁶O light water isotopologue more than 99.76% are also unknown from the art.

As a result of the increasing into the water of the content of light water isotopologue ¹H₂¹⁶O more than 99.76%, the water becomes enriched with ¹H₂¹⁶O and more isotope homogenous substance. Thus, the purity of the water is severely raised.

Herein and after the definitions, «water with abundance of light water isotopologue ¹H₂¹⁶O more than 99.76%», «water with increased content of ¹H₂¹⁶O» and «water enriched with ¹H₂¹⁶O» are used as convertible terms.

It is well-documented that heavy and light isotopologues of water affect distinctly on basic properties of substances that can be used in the cosmetic composition (proteins, carbohydrates, lipids, nucleic acids, and small molecules) (Chervenak et al. JACS, 1994, 116 (23): 10533-10539. Makhatadze et al., Nature Struct. Biol., 1995, 2 (10): 852-855. Connelly et al., PNAS, 1994, 91: 1964-1968. Cupane et al., Nucleic Acids Res. 1980, 8 (18): 4283-4303). Accordingly, the isotope heterogeneity of water is undesirable. In order to produce cosmetic composition with more reproducible properties the usage of monoisotopologous water with increased content of ¹H₂¹⁶O is more preferably.

As mention above, there is a great need for increasing of effectiveness and quality of the cosmetic compositions for maintain skin nutrition and appearance. Accordingly the present invention, water with increased content of ¹H₂¹⁶O is a safe, effective ingredient for improving of the cosmetic compositions quality.

It is an object of the present invention to provide a cosmetic composition comprising water with abundance of light water isotopologue ¹H₂¹⁶O more than 99.76%, i.e. cosmetic composition enriched with ¹H₂¹⁶O by weight of water of cosmetic composition.

US-A-5788953 discloses hygienic and cosmetic preparations for preventing and treating skin-diseases, comprising water having a lowered deuterium content of 111 to 135 ppm.

JP-A-2004/175775 discloses an aqueous solution comprising water having a deuterium content of 25 to 135 ppm with a carrier or an adjuvant.

JP-A-2004/231627 discloses a method of treating wrinkles, dullness, protuberances, skin cancer or red birth marks using a poultice having a pharmaceutical product or solution containing water, deuterium reduced water having a deuterium content of 149 ppm or less and an activator and carrier or adjuvant.

JP-A-2004/189708 discloses that periodontal disease may be prevented in humans by brushing teeth with a tooth powder containing deuterium reduced water having a deuterium content of less than 149 ppm.

### Brief Description of the Drawings

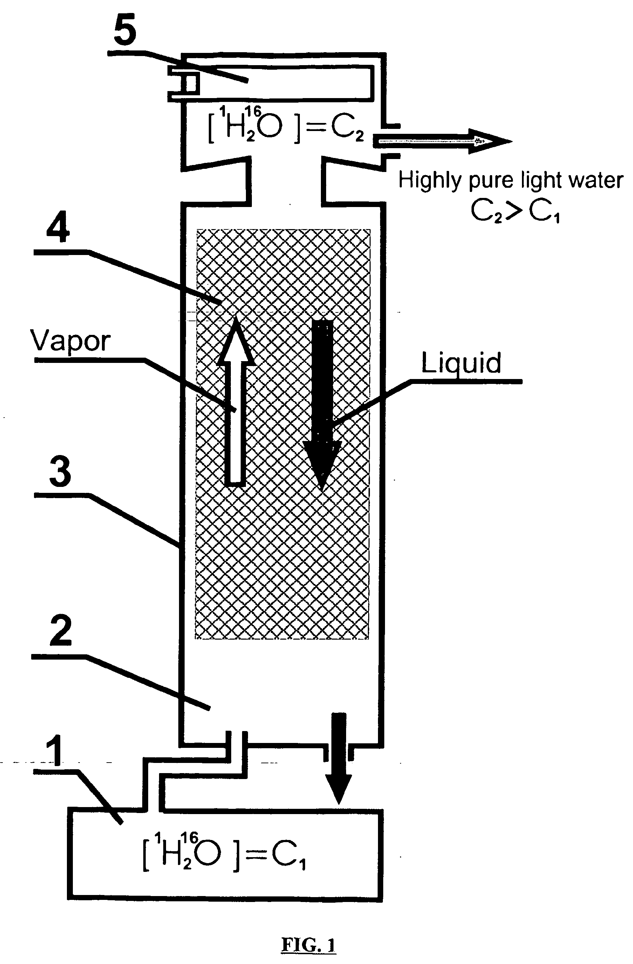

FIG.1 is a schematic side view of an apparatus for the production of water comprising from 99.76% to 99.999% of ¹H₂¹⁶O by weight.

### Disclosure of Invention

The present invention provides a cosmetic composition enriched with ¹H₂¹⁶O, wherein the content of ¹H₂¹⁶O is no less than 99.76% by weight of water of said cosmetic composition

Preferably, the present invention provides a cosmetic composition enriched with ¹H₂¹⁶O, wherein the content of ¹H₂¹⁶O is no less than 99.80% by weight of water of said a cosmetic composition.

Furthermore, the cosmetic composition of of the present invention provides a cosmetic composition comprising cosmetic remedy, hygienic remedy, perfumed remedy, cosmetic makeup, water for washing, water for bath.

The aim of the present invention was to develop cosmetics enriched with ¹H₂¹⁶O. To solve this problem typical water with typical content ¹H₂¹⁶O (from 99.731% to 99.757 % by weight of water) is substituted for water with increased content of ¹H₂¹⁶O in any cosmetic composition.

Herein and after the term «typical water» means any water with content of ¹H₂¹⁶O within the limits of VSMOW-SLAP standards, i.e. from 99.731% to 99.757 % by weight of water.

Except water, other components remain the same according to typical composition for conventional methods of the production of one or another cosmetic composition.

According to the present invention it is possible to produce water enriched with ¹H₂¹⁶O in an amount more than 99.76 % and up to 99.999% by weight of water. Water can be purified not only of typical chemicals and admixtures, but also of molecules, such as: ¹H₂¹⁷O, ¹H₂¹⁸O, ¹H²H¹⁶O, ¹H²H¹⁷O, ¹H²H¹⁸O, ¹H₂¹⁶O, ²H₂¹⁷O, ²H₂¹⁸O, which can amount up to 2.97 g/l and are a kind of admixtures concerning the main component of water, which is ¹H₂¹⁶O. As a result, the water becomes isotope homogenous substance consisting of ¹H₂¹⁶O in an amount up to 99.999 %, in other words, light water. This light water is pure water to a greater extent than any other purified water with typical isotope composition, it is highly pure light water. Thus, one can reach a qualitatively new and higher level of the water purity.

Thus, highly pure light water is a composition comprising from 99.76 % to 99.999% of ¹H₂¹⁶O and residual amounts of ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O up to 100% correspondingly.

Herein and after, the term "highly pure light water" refers to water comprising from 99.76% to 99.999% of the most light isotope variety of water molecules, lettering ¹H₂¹⁶O.

For practicing the invention we offer a method and apparatus for production of highly pure light water.

Highly pure light water comprising more than 99.76 % of light isotopologue ¹H₂¹⁶O is prepared by distillation of typical water with typical content ¹H₂¹⁶O with using the apparatus of FIG.1. It is prepared by methods providing simultaneous depletion from typical water of 8 isotope varieties of water molecules comprising heavy isotopes ²H, ¹⁷O, and ¹⁸O such as: ¹H₂¹⁷O, ¹H₂¹⁸O, ¹H²H¹⁶O, ¹H²H¹⁷O, ¹H²H¹⁸O, ²H₂¹⁶O, ²H₂¹⁷O, ²H₂¹⁸O.

The process of the distillation includes:
- evaporating natural water comprising [C₁] of light isotopologue ¹H₂¹⁶O in boiling (see FIG 1.1) to produce water vapor;
- supplying the water vapor to the bottom (2) of distillation column (3);
- carrying out vapor-liquid contact between a descending liquid and an ascending vapor mainly on the surface of the contact device (4) (e.g. structured or random packing) within the distillation column, simultaneously the liquid and the vapor flow in mutually opposite directions over the surface of the contact device along the main flow direction which is the direction of the column axis;
- condensing water vapor with concentration of light isotopologue ¹H₂¹⁶O [C₂] on condenser (5) installed on the top of the distillation column;
- collecting a part of condensate as condensed highly pure light water comprising the increased content (more than 99.76 %) of light isotopologue ¹H₂²⁶O[C₂ > C₁].

After respective treatment one can get highly pure light water which is distilled water, deionized water, reverse osmosis water, ultra-pure water, drinking water, etc. These types of water differ by concentration of chemical substances, but it always comprises more than 99.76% of light isotopologue ¹H₂¹⁶O.

Highly pure light water in the cosmetic composition acts as a dilutant, dispersant or carrier independently or in addition to vehicles other than water. Also, in cosmetic composition a highly pure light water acts as a moistening, cleansing etc. ingredient.

The total quantity of water in a cosmetic composition as component or base can amount from 0.1% to 99.9 % by weight of cosmetic composition and a physiologically acceptable cosmetic base or component can amount from 99.9 % to 0.1 % by weight of said cosmetic composition, correspondingly. Hence, even if the quantity of water in a cosmetic composition amounts only 0.1 % by weight of said cosmetic composition, all this water can be highly pure light water. In other words, in that case highly pure light water amounts 100% by weight of total water of said cosmetic composition.

As used herein, the term «base» means that water, cosmetic substance or their mixture amounts more than 50 % by weight of cosmetic composition. Highly pure light water can be both component and base. Also highly pure light water can be part of typical complicated cosmetic bases. Such bases can be formulate so that they are compositions comprising some of the necessary standard ingredients for manufacturing of a definite product such as cream, shampoo, soap, etc. Then specific essential ingredients can be added directly to base for manufacturing of the finished product.

Nonexclusive examples of cosmetic base include cream base, liquid soap base, lotion base, shampoo base, etc.

Nonexclusive examples of liquid soap base include highly pure light water, sodium laureth sulphate, glycerine, cocamidopropyl betaine, sodium laureth sulfate, glycol distearate, cocamide MEA, laureth 10, sodium chloride, cocamide DEA, coconut oil, phenoxyethanol, methyl parabens, citrica acid. Different fragrances can be added to this liquid soap base.

As used herein, the term «cosmetic remedy» includes cosmetic products for care, nutrition, maintenance of appearance of skin, hair, nail, teeth and oral cavity.

As used herein, the term « hygienic remedy» includes hygienic products for skin, hair, nail, teeth and oral cavity.

As used herein, the term «perfumed remedy» includes cologne, toilet water, etc. and also any perfumed cosmetic or hygienic products.

As used herein, the term «cosmetic make up» refers to products that leave color on the face, blacks and browns, i.e., mascara, concealers, eye liners, brow colors, eye shadows, blushers, lip colors, powders, solid emulsion compact, etc., and also foundation and base for make up.

As used herein, the terms «water for washing» and «water for bath» mean highly pure light water in the absence of the other cosmetic ingredient as cosmetic and/or hygienic product.

The cosmetic composition of the present invention except highly pure light water and obligatory components includes, but are not limited to, other optional components selected from the group consisting depigmentation, clarification, moistening, softening, astringent, anti-aging, antiwrinkle, lifting, UV- adsorbent agents, agents for healing of a wound, antiseptic, topical anesthetic agents, antimicrobe, antibacterial, antimycotic, antiacne agents, deodorant and antiperspirant agents, depilation agents, herbal extract and, retinoids, bioflavonoids, antioxidants, hair conditioner, hair decolorants, chelating agents, cell reparation stimulators, colorant agents, sunblock agents, amino acids and their derivations, vitamins, micro and macroelements, etc., or mixture thereof.

The vitamins are selected from the group consisting thiamine, riboflavin, niacin, pantothenate, pyridoxine, folic acid, cobalamin, biotin, choline, inositol, ascorbic acid, lipoic acid, carnitine, etc., or mixture thereof.

The micro and macroelements are selected from the group consisting boron, calcium, chromium, cobalt, copper, fluorine, germanium, iodine, iron, lithium, magnesium, manganese, molybdenum, phosphorus, natrium, selenium, silicon, potassium, sulfur, vanadium, zinc, etc., or mixture thereof.

The amino acids and their derivations are selected from the group consisting alanine, arginine, aspartame acid, citrulline, cystine, dimethylglycine, glutamic acid, glutamate, glutathione, glycine, histidine, isoleucine, leucine, lysine, methionine, ornithine, phenylalanine, prolin, serine, taurine, threonine, tryptophan, tyrosine, valine, etc., or mixture thereof.

The cosmetic composition of the present invention are used for a face, neck, body, hands, legs, foots skincare; hair-care and head's skincare, nails-care, tooth-care, oral cavity-care; for a face, eyebrows, eyelashes, lips, body, hands, legs care; for all age groups, for all skin and hair types, etc.

The cosmetic composition of the present invention are used as liposome agents, for a prevention and removal of wrinkles, skin sulci, skin surface imperfections, skin chaps, skin texture changes, for a skin lifting, skin elasticity increasing, for askin flabbiness and area under eyes swelling elimination, for a skin oedema, double chin and cheeks flabbiness removal, for a correction of face oval, for a prevention and removal skin thinning, for collagen reparation, for a removal other histological changes in the hypodermic cellular tissue, supplying the skin, in the skin basal layer, corium, epidermis, skin circulatory system such as haemorrhage, for a prevention and removal the cellulitis, for a reparation of a skin elastin, skin, hair, nails elasticity, for a removal hair, nails fragility, for a removal nail plate defects, for the a skin clarification, skin colour and complexion improvement, for a removal excessive pigmentation, age and other nevuses pigmentosis, for an improving of metabolic processes in a skin, hair, nails and oral cavity mucous membrane, etc.;
for a refreshing, moistening, softening, nutrition and reparation skin, hair, nails, oral cavity mucous membrane, for an elasticity improvement, for a removal different skin, hair, nails, oral cavity mucous membrane disturbances, as visible so tactile, etc.;
for a removal a disturbances of the function of sebaceous glands, sudoriferous glands, lymphatic glands, skin peeling, seborrhea and other skin disturbances, including skin of head; for prevention and removal dandruff, different types of acne, etc. The examples are illustrative only and are not intended to limit the scope of the invention in any way.

The cosmetic composition of the present invention are used before shaving, after shaving, to shaving; before epilation, after epilation, to epilation; to sunburn, against sunburn; before sun-bath, hydro procedure, sport training; during sun-bath, hydro procedure, sport training; after sun-bath, hydro procedure, sport training; to correction figure and weight loss, etc.;
as a neuromuscular relaxant; hairset, haircut remedy; manicure, pedicure remedy; removal makeup remedy; to dyeing of hair, eyebrows, eyelashes, lips, cheeks, nails, to coloring of cheeks, face and body skin; for removal nail polish, etc.;
to a prevention and removal gingival hemorrhage and reinforcement oral cavity mucous membrane, to a prevention and removal irritation and dermatitis in oral cavity, to a removal foul smell, to a tooth brushing and dental bleaching, etc.;
as a disinfectant, cleanser, protective remedy, refreshing remedy, antiinflammatory remedy, stanch remedy, deodorant, antiperspirant, intimate hygiene remedy, to a prevention and a removal sting consequences, to aromatization, etc. The examples are illustrative only and are not intended to limit the scope of the invention in any way.

Cosmetic composition of the invention are available in the form of lotion, tonic, spray, gel, jelly, suspension, emulsion, milk, froth, serum, cream, ointment, mask, scrub, liquid wax, balm, rinsing, conditioning remedy, coloring or dis coloring remedy, haircut fixator, hair mask, lathering composition, makeup foundation, makeup base, liquid powder, tonal cream, liquid shades, blush, lipstik, hair-dye, lips-dye, eyebrows-dye, eyelashes-dye, nail polish and removal polish composition, hair spray, oral rinsing, tooth elixir, shampoo, solid toilet and liquid soap, detergent, cologne, toilet water, etc. The examples are illustrative only and are not intended to limit the scope of the invention in any way.

Cosmetic composition of the invention is produced by conventional methods with usage different technological additives which are necessary for production of cosmetic remedies, hygienic remedies, perfumed remedies, cosmetic makeup. Nonexclusive examples of technological additives include stabilizer, preservative, emulsifier, acidulant, gelling agent, thickener, solvent, dilutant, dispersant, carrier, surfactant, fragrance, colorant, absorption promoter, etc. or mixture thereof. The examples are illustrative only and are not intended to limit the scope of the invention in any way.

As mention above, water with typical content ¹H₂¹⁶O (from 99.731% to 99.757 % by weight of water) can be substituted for highly pure light water enriched with ¹H₂¹⁶O in an amount no less than 99.76% in any cosmetic composition. Except water, other components remain the same according to typical composition for conventional methods of the production of one or another cosmetic composition. All physiologically acceptable additives are used in forms, which are necessary for production one or another cosmetic composition. Such components may be dispersed, solubilized, or otherwise mixed into the present cosmetic compositions. Several solid ingredients may be dissolved in highly pure light water or in hot highly pure light water if required prior to addition to the other components.

It is necessary mention, a content of highly pure light water into finished product can be insignificant, at the same time, highly pure light water was used in a significant volume at the time of producing process of this product, for example, solid soap.

It is also necessary mention that the total quantity of water in a cosmetic composition of the present invention can comprise not only highly pure light water, but some quantity of typical water which is in solid or liquid ingredients for producing one or another cosmetic composition. Thus, the final content of ¹H₂¹⁶O in the total quantity of water into a cosmetic composition is determined taking for account this factor.

In practicing the method of the invention, the topical composition of the invention in a unit dosage form is applied topically to the skin, hair, nail, teeth and oral cavity of human, and is preferably left on the surface of skin, hair, nail, etc., for a period of at least 5 minutes, more preferably at least 30 minutes, even more preferably at least 1 hour, most preferably for at least several hours, e.g., up to 12 hours. Typically, the effective amount of the composition is from 1 gram to 10 grams, preferably from 1 gram to 2 grams. This method can be reapplied from 1 to 5, preferably from 1 to 3 times per day. Also, cosmetic composition of the invention can be use in an injection mezotherapy.

Any cosmetic composition comprises water as a universal, the most physiologically acceptable solvent, diluter or carrier. The isotope homogeneity of water consisting of cosmetic composition of the present invention provides particularly advantageous and effective cosmetic compositions. Cosmetic compositions of the present invention have more reproducible and stable properties. Consequently, the present invention provides the improving of consumer qualities of the different cosmetic compositions. In practicing this invention, it is possible to minimize potential adverse effects arising from diversity of water isotopologues.

Cosmetic compositions enriched with ¹H₂¹⁶O in an amount no less than 99.76% by weight of water of cosmetic composition, which are obtained by the present invention, are a new product which has not been produced up to this time.

Cosmetic composition enriched with ¹H₂¹⁶O in an amount no less than 99.76 % by weight of water of cosmetic composition are composition with best quality, because they offer all advantages of light water. The volunteers registered improved properties of the cosmetic compositions enriched with ¹H₂¹⁶O.

The following examples are presented to demonstrate the invention. The examples are illustrative only and are not intended to limit the scope of the invention in any way.

### Example 1

This example demonstrates the method for producing highly pure light water of the invention.

Light water comprising 99.90% of light isotopologue ¹H₂¹⁶O is prepared by distillation of natural water comprising 99.70% of light isotopologue ¹H₂¹⁶O with using the apparatus in FIG.1 1 under temperature 60°C and pressure 20KPa (0.2 bars). The process of the distillation includes:
- evaporating natural water comprising 99.70% [C₁] of light isotopologues ¹H₂¹⁶O in boiling (see Fig 1.1) to produce water vapor;
- supplying the water vapor to the bottom (2) of distillation column (3);
- carrying out vapor-liquid contact between a descending liquid and an ascending vapor mainly on the surface of the contact device (4) (e.g. structured or random packing) within the distillation column, simultaneously the liquid and the vapor flow in mutually opposite directions over the surface of the contact device along a main flow direction which is along a direction of the column axis;
- condensing water vapor with concentration of light isotopologue ¹H₂¹⁶O 99.90% [C₂] on condenser (5) installed on upper bound of the distillation column;
- and collecting a part of condensate as condensed highly pure light water comprising 99.90% of light isotopologue ¹H₂¹⁶O [C₂ > C₁] appropriate for producing cosmetic composition enriched with ^{I}H₂¹⁶0.

### Example 2

This example demonstrates a representative cosmetic composition enriched with ¹H₂¹⁶O for prevention unfortunate effects of ageing.

| Ingredients | %, Weight |
|---|---|
| Highly pure light water enriched with ¹H₂¹⁶O, wherein the content of ¹H₂¹⁶O is 99.85 % by weight of water | 97 |
| Ascorbic acid | 1 |
| Vitamin B5 | 1 |
| Vitamin B6 | 1 |

The final content of ¹H₂¹⁶O in the water of lotion enriched with ¹H₂¹⁶O amounts no less than 99.846 % under the most exacting requirement, that quantity of typical water with the least content of ¹H₂¹⁶O 99.731 % amounts up to 3 % by weight of total lotion water.

The vitaminous cosmetic anti-aging composition enriched with ¹H₂¹⁶O is prepared by conventional manner. The pH was adjusted to pH 5.5.

### Example 3

This example demonstrates a representative application of the cosmetic anti-aging composition enriched with ¹H₂¹⁶O.

The composition described in Example 2 is applied topically to the human skin from 1 to 3 times per day and can be leave on the skin for a period up to 12 hours.

### Example 4

This example demonstrates a representative topical cosmetic composition of skin moistening lotion enriched with ¹H₂¹⁶O.

| Ingredients | %, Weight |
|---|---|
| Highly pure light water enriched with ¹H₂¹⁶O, wherein the content of ¹H₂¹⁶O is 99.85 % by weight of water | 97.5 |
| Glycerin | 2.5 |

The final content of ¹H₂¹⁶O in the water of the topical cosmetic composition enriched with ¹H₂¹⁶O amounts no less than 99.847 % under the most exacting requirement, that quantity of typical water with the least content of ¹H₂¹⁶O 99.731 % amounts up to 2.5 % by weight of the total cosmetic composition water.

The method for preparing the composition described in Example 3 was as follows: the glycerin and highly pure light water were mixed.

### Example 5

This example demonstrates a representative formulation for skin face gel enriched with ¹H₂¹⁶O for prevention of dermatitis.

| Ingredients | %, Weight |
|---|---|
| Triethanolamine | 0.4 |
| Propylene glycol | 3.5 |
| Hydroxyethylcellulose | 0.6 |
| Carbopol 940 | 0.4 |
| Glycerin | 2.5 |
| Calendula aqueous-alcoholic extract | 1.4 |
| Camomile aqueous-alcoholic extract | 1.3 |
| Nettle aqueous-alcoholic extract | 1.3 |
| Highly pure light water enriched with ¹H₂¹⁶O, wherein the content of ¹H₂¹⁶O is 99.88 % by weight of water | Up to 100 |

The final content of ¹H₂¹⁶O in the water of the gel enriched with ¹H₂¹⁶O amounts no less than 99.862 % under the most exacting requirement, that quantity of typical water with the least content of ¹H₂¹⁶O 99.731 % amounts up to 12 % by weight of the total gel composition water.

The method for preparing the composition described in Example 5 was as follows: all components excepting gelling agents was mixed in a necessary proportion; the gelling agents was dispersed in the highly pure light water enriched with ¹H₂¹⁶O, wherein the content of ¹H₂¹⁶O is 99.88 % by weight of water, at 150 °C; after the formation of homogeneous dispersion obtained gel was neutralized and added with mixing to the rest components; the mixture was chilled to room temperature, filtered and after then it was packed.

### Example 6

This example demonstrates a representative formulation for body cream enriched with ¹H₂¹⁶O for moistness of body skin.

| | Ingredients | Weight, g |
|---|---|---|
| 1. | Dimethyl polysiloxane (6 mPa .multidot. s) | 10.0 |
| 2. | Decamethyl cyclopentasiloxan | 6.0 |
| 3. | 1,3-butylene glycol | 3.0 |
| 4. | Paste-like polyether-modified silicone | 2.5 |
| 5. | 50% solution of octamethyl cyclotetrasil oxane of polyether-modified silicone composition | 2.5 |
| 6. | Paraben | 0.2 |
| 7. | Antioxidant | 0.1 |
| 8. | Ethanol | 3.0 |
| 9. | Fragrance | 0.1 |
| 10. | Deionized highly pure light water enriched with ¹H₂¹⁶O, the content of ¹H₂¹⁶O is 99.88 % by weight of water | 72.6 |

The final content of ¹H₂¹⁶O in the water of the body cream enriched with ¹H₂¹⁶O amounts no less than 99.859 % under the most exacting requirement, that quantity of typical water with the least content of ¹H₂¹⁶O 99.731 % amounts up to 14 % by weight of the total body cream composition water.

The method for preparing the composition described in Example 6 was as follows: (1), (2), (4), and (5) were mixed to prepare an oil phase mixture in advance; next, while stirring this oil-phase mixture using a high speed agitator, an aqueous phase mixture obtained by mixing and stirring (3) and (6) to (10) to dissolve was gradually added at room temperature to obtain the body cream.

### Example 7

This example demonstrates a representative formulation for nail lotion enriched with ¹H₂¹⁶O for the improvement of nails growth.

| Ingredients | %, Weight |
|---|---|
| Ethyl alcohol | 35 |
| Mineral kelp concentrated product | 6.5 |
| Copper salt of chlorophyll | 0.55 |
| Aspen bark aqueous- extract | 0.25 |
| Abietene | 0.25 |
| Collagen | 0.25 |
| Limonite | 0.25 |
| Epidermis softener | 0.25 |
| Highly pure light water enriched with ¹H₂¹⁶O, wherein the content of ¹H₂¹⁶O is 99.88 % by weight of water | Up to 100 |

The final content of ¹H₂¹⁶O in the water of the nail lotion enriched with ¹H₂¹⁶O amounts no less than 99.847 % under the most exacting requirement, that quantity of typical water with the least content of ¹H₂¹⁶O 99.731 % amounts up to 45 % by weight of the total nail lotion composition water.

The nail lotion for the improvement of nails growth enriched with ¹H₂¹⁶O is prepared by conventional manner.

### Example 8

This example demonstrates a representative formulation for anti-irritant shampoo enriched with ¹H₂¹⁶O.

| Ingredients | Weight, kg |
|---|---|
| Monoethanolamides | 0.5 |
| Oxyethylated lauryl sulphate | 2.0 |
| Calendula hydro phyto concentrated product | 0.005 |
| Citric acid | 0.001 |
| Resorcin | 0.05 |
| Odorant | 0.13 |
| Dye | 0.00025 |
| Sodium chloride | 0.5 |
| Highly pure light water enriched with ¹H₂¹⁶O, wherein the content of ¹H₂ ¹⁶O is 99.80 % by weight of water | 7.8 |

The final content of ¹H₂¹⁶O in the water of the shampoo enriched with ¹H₂¹⁶O amounts no less than 99.783 % under the most exacting requirement, that quantity of typical water with the least content of ¹H₂¹⁶O 99.731 % amounts up to 15 % by weight of the total shampoo composition water.

For producing of the shampoo the highly pure light water enriched with ¹H₂¹⁶O, wherein the content of ¹H₂¹⁶O is 99.80 % by weight of water, in an amount 7.8 kg was heated up to 70 C in the digeer. The preliminary molten monoethanolamides in an amount 0.5 kg was added with mixing and heating to the highly pure light water in the digeer. After the monoethanolamides was completely dissolved the oxyethylated lauryl sulphate was slowly, a little at a time, added into the digeer in an amount 2.0 kg with stirring. Then, the preliminary dissolved in a quantity of highly pure light water calendula hydro phyto concentrated product was added in an amount 0.005 kg. After the cooling of the mixture up to 45-50° C the citric acid, resorcin, odorant, dye, sodium chloride were added in an amount 0.001 kg, 0.05 kg, 0.13 kg, 0.00025 kg, 0.5 kg, correspondingly. After the cooling total mixture up to room temperature the shampoo is ready.

### Example 9

This example demonstrates a representative formulation for tooth-paste enriched with ¹H₂¹⁶O including fluorine.

| Ingredients | %, Weight |
|---|---|
| Highly pure light water enriched with ¹H₂¹⁶O, wherein the content of ¹H₂¹⁶O is 99.95 % by weight of water | 42.5 |
| Aluminium hydroxide | 10 |
| Hydrated silica | 3 |
| Silicon dioxide | 10 |
| Sodium chloride | 10 |
| Glycerin | 10 |
| Sorbitol | 10 |
| Carboxymethylcellulose | 1 |
| Sodium lauryl sulfate | 2 |
| Sodium fluoride | 0.5 |
| Aroma | 1 |

The final content of ¹H₂¹⁶O in the water of the tooth-paste enriched with ¹H₂¹⁶O amounts no less than 99.862 % under the most exacting requirement, that quantity of typical water with the least content of ¹H₂¹⁶O 99.731 % amounts up to 40 % by weight of the total tooth-paste composition water.

The method for preparing the tooth-paste including fluorine described in Example 9 was as follows: listed ingredients were mixed by conventional manner up to homogeneous mass.

### Example 10

This example demonstrates a representative formulation for oral cavity refreshing gel enriched with ¹H₂¹⁶O.

| Ingredients | %, Weight |
|---|---|
| Highly pure light water enriched with ¹H₂¹⁶O, wherein the content of ¹H₂¹⁶O is 99.95 % by weight of water | 58.13 |
| Sorbitol (70%) | 34 |
| Glycerin | 5 |
| Carbopol 940 | 1 |
| Sodium hydroxide | 0.85 |
| Aroma | 0.40 |
| Citric acid | 0.17 |
| Sorbic acid | 0.15 |
| Methylparaben | 0.15 |
| Crystallose | 0.10 |
| Propylparaben | 0.05 |

The final content of ¹H₂¹⁶O in the water of the oral cavity gel enriched with ¹H₂¹⁶O amounts no less than 99.884 % under the most exacting requirement, that quantity of typical water with the least content of ¹H₂¹⁶O 99.731 % amounts up to 30 % by weight of the total oral cavity gel composition water.

The method for preparing the composition described in Example 10 was as follows: highly pure light water enriched with ¹H₂¹⁶O, wherein the content of ¹H₂¹⁶O is 99.95 % by weight of water, sorbitol, aroma, citric acid, methylparaben and crystallose in the ratio specified in the table were mixed and heated up to 60°C; then glycerin in an amount 5.00 g was added under stirring; then also under stirring the carbopol 940 in an amount 1.00 g was added and the pH was adjusted to pH 5.5.

### Example 11

This example demonstrates a representative formulation for water for washing enriched with ¹H₂¹⁶O.

| Ingredients | Content, weight % |
|---|---|
| Distilled highly pure light water enriched with ¹H₂¹⁶O, wherein the content of ¹H₂¹⁶O is 99.80% by weight of water | 99.953 |
| Calcium chloride | 0.015 |
| Magnesium chloride | 0.007 |
| Sodium bicarbonate | 0.025 |

The final content of ¹H₂¹⁶O in the water for washing is 99.80% by weight of water.

The method for preparing the water for washing enriched with ¹H₂¹⁶O described in Example 11 was as follows: components such as bicarbonate and chloride salts of calcium, magnesium, and sodium were dissolved in light water at room temperature.

### Example 12

This example demonstrates a representative formulation for mascara enriched with ¹H₂¹⁶O.

| Ingredients | %, Weight |
|---|---|
| Carnuba wax | 3.00 |
| glycerol monostearate | 7.50 |
| White beeswax | 3.75 |
| C18-C36 Triglycerides | 5.50 |
| Hydrogenated glycerol rosinate | 0.15 |
| Propylparaben | 0.10 |
| Paraffin wax 118/125 | 2.25 |
| Paraffin-wax | 2.35 |
| Elastomer gel (KSG21). | 2.31 |
| DC9040 elastomer gel | 15.00 |
| Lecithin | 2.25 |
| Stearic acid 3X | 4.00 |
| Oleic acid | 0.75 |
| Triethanolamine | 1.72 |
| Potassium cetyl phosphate | 1.00 |
| Shellac, NF | 3.00 |
| Trisodium EDTA | 0.10 |
| Black iron oxide | 7.00 |
| Simethicone | 0.20 |
| Methylparaben | 0.20 |
| Ethylparaben | 0.15 |
| Phenoxyethanol | 0.80 |
| Ethyl alcohol | 4.00 |
| Diazolidinyl urea | 0.20 |
| Deionized highly pure light water enriched with ¹H₂¹⁶O, wherein the content of ¹H₂¹⁶O is 99.95 % by weight of water | 30.22 |
| dl-Panthenol | 0.35 |
| Niacinamide | 2.25 |
| Total | 100 |

The final content of ¹H₂¹⁶O in the water of mascara composition enriched with ¹H₂¹⁶O amounts no less than 99.832 % by weight of the total mascara composition water.

For producing the mascara enriched with ¹H₂¹⁶O described in Example 12 the waxes and fats are mixed in a vessel equipped with a heating source. The waxes and fats are heated and mixed at low speed using a conventional blender to liquefy the mixture. The mixing is continued until the mixture is homogeneous. To the homogenous mixture is added the pigments. The mixing rate is increased to high and the pigments are mixed into the mixture for 30-35 minutes until uniformly dispersed. The mixing is continued while adding emulsifiers.

In a second vessel equipped with a heating source is added highly pure light water followed by the niacinamide, lecithin and any other water-dispersible components. The mixture is heated and mixed to a temperature of from 80-95.degree. C. Additional highly pure light water is added as necessary to account for water loss.

The aqueous and lipophilic mixtures are combined and mixed using a dispersator type mixer. Mixing is continued until the mixture cools to a temperature of from 65-70. degree. C. Elastomer gels and preservatives are added with mixing, allowing the mixture to cool further to 45-47.degree. C. Any remaining components are added with stirring. The combined mixture is cooled to a temperature above the solidification point and is then poured into suitable containers.

### Example 13

This example demonstrates a representative formulation for lipstick enriched with ¹H₂¹⁶O.

| Ingredients | Weight, g |
|---|---|
| Aqueous dispersion of styrene-acrylate polymer (45%) | 50.00 |
| Yellow-brown iron oxide | 4.00 |
| Preservatives | 0.25 |
| Acrylic gelling agent | 5.00 |
| Polyethylene/polytetrafluoroethylene wax (50/50) | 4.50 |
| Ethyl alcohol | 5.00 |
| Additives | 3.00 |
| Deionized highly pure light water enriched with ¹H₂¹⁶O, wherein the content of ¹H₂¹⁶O is 99.95 % by weight of water | q.s. for 100 |

The final content of ¹H₂¹⁶O in the water of lipstick composition enriched with ¹H₂¹⁶O amounts no less than 99.832 % by weight of the total lipstick composition water.

The lipstick composition enriched with ¹H₂¹⁶O prepared by conventional manner.

### Example 14

This example demonstrates a representative formulation for liquid foundation enriched with ¹H₂¹⁶O.

| Ingredients | %, Weight |
|---|---|
| Cyclomethicone | 11.62 |
| Dimethicone copolyol emulsifier | 0.70 |
| KSG32 Elastomer Gel. sup.1 | 5.38 |
| GE SFE839 Elastomer gel. sup.2 | 10.00 |
| Isononyl isononanoate | 5.00 |
| n-Propyl-4-hydroxybenzoic Acid | 0.20 |
| Ethylene brassylate | 0.03 |
| Titanium dioxide | 17.8 |
| Yellow iron oxide | 1.70 |
| Red iron oxide | 0.19 |
| Black iron oxide | 0.11 |
| Methylparahydroxybenzoate | 0.12 |
| Glycerin | 10.00 |
| 2-amino-2-methyl-1-propanol | 0.10 |
| Deionized highly pure light water enriched with ¹H₂¹⁶O, wherein the content of ¹H₂¹⁶O is 99.95% by weight of water | 36.45 |
| Sucrose oleate ester | 0.60 |
| Total | 100 |

sup.1- 25% Lauryl Dimethicone/Copolyol Crosspolymer in isododecane; sup.2- 5% Dimethicone/Vinyl Dimethicone crosspolymer (aver. particle sizeof at least 20 microns) in cyclomethicone.
The final content of ¹H₂¹⁶O in the water of liquid foundation composition enriched with ¹H₂¹⁶O amounts no less than 99.830 % by weight of the total liquid foundation composition water.

For producing the liquid foundation enriched with ¹H₂¹⁶O described in example 14 in a suitable stainless steel vessel, the cyclomethicone, dimethicone copolyol, GE SFE 839, KSG32, isononyl isononanoate, n-propyl-4-hydroxybenzoic acid, and ethylene brassylate are added with mixing using conventional mixing technology and mixed until homogeneous. In a separate vessel equipped with a heat source, the sucrose oleate ester and highly pure light water are heated to 50.degree. C. and mixed using conventional mixing technology until homogeneous. The sucrose oleate ester mixture is then allowed to cool to room temperature. Once cooled, the titanium dioxide, iron oxides, methylparahydroxy benzoate, glycerin and 2-amino-2-methyl-1-propanol are added to sucrose oleate ester mixture with mixing to form homogeneous, pigment slurry. Next, the sucrose oleate ester mixture is combined with the cyclomethicone mixture and mixed using conventional mixing technology until homogeneous. The combined mixture is then poured into suitable containers.

### Example 15

This example demonstrates a representative formulation for non-alcoholic perfume or cologne enriched with ¹H₂¹⁶O.
The following ingredients were blended in a conventional manner in the indicated amounts.

| Ingredients | Weight, g |
|---|---|
| Fragrance oil | 5.00 |
| Pelemol G-7A | 5.50 |
| Cremophor RH-60 | 3.25 |
| Arlasolve 200L | 2.0 |
| Tergitol NP-13 | 1.0 |
| Propylene glycol | 12.25 |
| Germaben II | 1.10 |
| Deionized highly pure light water enriched with ¹H₂¹⁶O, wherein the content of ¹H₂¹⁶O is 99.95 % by weight of water | 69.90 |

The final content of ¹H₂¹⁶O in the water of non-alcoholic perfume composition enriched with ¹H₂¹⁶O amounts no less than 99.911 % by weight of the total non-alcoholic perfume composition water.

## Claims

1. A cosmetic composition enriched with ¹H₂¹⁶O, wherein the content of ¹H₂¹⁶O is no less than 99.76% by weight of water of said cosmetic composition.

2. The cosmetic composition of claim 1, wherein the content of ¹H₂¹⁶O is no less than 99.80% by weight of water of said cosmetic composition

3. The cosmetic composition of claims 1-2, wherein said cosmetic composition is selected from the group comprising cosmetic remedy, hygienic remedy, perfumed remedy, cosmetic makeup, water for washing, water for bath.

## Patentansprüche

1. Kosmetische Zusammensetzung angereichert mit ¹H₂¹⁶O, wobei der Gehalt an ¹H₂¹⁶O nicht geringer ist als 99,76 Gewichts-% des Wassers der kosmetischen Zusammensetzung.

2. Kosmetische Zusammensetzung nach Anspruch 1, wobei der Gehalt an ¹H₂¹⁶O nicht geringer ist als 99,80 Gewichts-% des Wassers der kosmetischen Zusammensetzung.

3. Kosmetische Zusammensetzung nach Ansprüchen 1-2, wobei die kosmetische Zusammensetzung ausgewählt ist aus der Gruppe umfassend kosmetisches Mittel, hygienisches Mittel, parfümiertes Mittel, kosmetisches Make-up, Wasser zum Waschen, Wasser zum Baden.

## Revendications

1. Composition cosmétique enrichie en ¹H₂¹⁶O, dans laquelle la teneur en ¹H₂¹⁶O est au moins égale à 99,76% en poids d'eau de ladite composition cosmétique.

2. Composition cosmétique de la revendication 1, dans laquelle la teneur en ¹H₂¹⁶O est au moins égale à 99,80% en poids d'eau de ladite composition cosmétique.

3. Composition cosmétique des revendications 1 et 2, dans laquelle ladite composition cosmétique est choisie dans le groupe composé d'un remède cosmétique, d'un remède hygiénique, d'un remède parfumé, d'un maquillage cosmétique, de l'eau pour lavage, de l'eau pour le bain.
